(19) Europäisches Patentamt
European Patent Office
Office européen des brevets

(11) Veröffentlichungsnummer: **0 064 775**
**A1**

(12) # EUROPÄISCHE PATENTANMELDUNG

(21) Anmeldenummer: 82105942.5

(22) Anmeldetag: 01.08.80

(51) Int. Cl.³: **C 07 D 487/04**
//A61K31/55, C07D231/38, C07D231/16, C07D231/14, C07C59/185, (C07D487/04, 243/10, 231/10)

(30) Priorität: 03.08.79 CH 7128/79
10.08.79 CH 7333/79
23.04.80 CH 3149/80

(43) Veröffentlichungstag der Anmeldung:
17.11.82 Patentblatt 82/46

(84) Benannte Vertragsstaaten:
AT BE CH DE FR GB IT LI LU NL SE

(60) Veröffentlichungsnummer der früheren Anmeldung nach Art. 76 EPÜ: 0 023 707

(71) Anmelder: Byk Gulden Lomberg Chemische Fabrik GmbH
Byk-Gulden-Strasse 2
D-7750 Konstanz(DE)

(72) Erfinder: Rainer, Georg, Dr.
Josef-Anton-Feuchtmayer-Strasse 7
D-7750 Konstanz(DE)

(72) Erfinder: Riedel, Richard, Dr.
Salmannsweilergasse 36
D-7750 Konstanz(DE)

(72) Erfinder: Klemm, Kurt, Prof. Dr.
Im Weinberg 2
D-7753 Allensbach(DE)

(72) Erfinder: Eltze, Manfrid, Dr.
Schützenstrasse 20
D-7750 Konstanz(DE)

(54) **Substituierte Tetraazatricyclen, Verfahren zu ihrer Herstellung und ihre Verwendung.**

(57) Substituierte Pyrazolobenzodiazepinone der allgemeinen Formel I

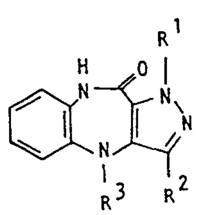

(I),

worin $R^1$ einen Alkylrest mit 1 bis 4 Kohlenstoffatomen, $R^2$ ein Wasserstoffatom oder einen Alkylrest mit 1 bis 4 Kohlenstoffatomen, $R^3$ ein Wasserstoffatom oder die Gruppe $-CO-A-R^4$, $R^4$ ein Halogenatom und A eine geradkettige oder verzweigte Alkylengruppe mit 1 bis 5 Kohlenstoffatomen bedeuten, und ihre Säureadditionssalze sind neue Verbindungen. Sie stellen Zwischenprodukte dar. Verfahren zur Herstellung der neuen Verbindungen sowie der Zwischenprodukte werden angegeben.

EP 0 064 775 A1

Croydon Printing Company Ltd

Die Erfindung betrifft Tetraazatricyclen, Verfahren zu ihrer Herstellung und ihre Verwendung.

Die erfindungsgemäßen Verbindungen werden in der pharmazeutischen Industrie als Zwischenprodukte zur Herstellung von Medikamenten verwendet (siehe europäische Patentanmeldung EP-OS 0 023 707).

Gegenstand der Erfindung sind substituierte Pyrazolobenzodiazepinone der allgemeinen Formel I

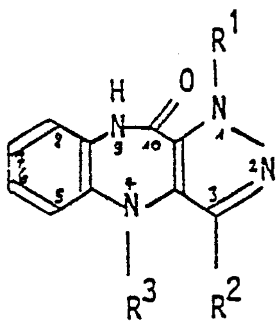

(I),

worin

$R^1$ einen Alkylrest mit 1 bis 4 Kohlenstoffatomen,

$R^2$ ein Wasserstoffatom oder einen Alkylrest mit 1 bis 4 Kohlenstoffatomen,

$R^3$ ein Wasserstoffatom oder die Gruppe $-CO-A-R^4$,

$R^4$ ein Halogenatom und

A eine geradkettige oder verzweigte Alkylengruppe mit 1 bis 5 Kohlenstoffatomen bedeuten,

sowie ihre Säureadditionssalze.

Alkylreste mit 1 bis 4 Kohlenstoffatomen sind der Methyl-, Ethyl-, Propyl-, Isopropyl-, n-Butyl-, Isobutyl-, sek.-Butyl-, tert.-Butylrest. Von den Alkylresten sind der Methyl- und Ethylrest bevorzugt. Besonders bevorzugt als Alkylrest $R^1$ und $R^2$ ist der Methylrest.

Halogenatome (Hal) sind das Iod-, das Brom- und insbesondere das Chloratom.

Alkylengruppen mit 1 bis 5 Kohlenstoffatomen sind die Trimethylen-, Tetramethylen-, Pentamethylen-, Propylen-, Ethylmethylengruppe, bevorzugt die Ethylengruppe, insbesondere die Methylengruppe.

Als Salze kommen alle Säureadditionssalze in Betracht. Besonders erwähnt seien die pharmakologisch verträglichen Salze der in der Galenik üblicherweise verwendeten anorganischen und organischen Säuren. Pharmakologisch unverträgliche Salze werden durch dem Fachmann bekannte Verfahren in pharmakologisch verträgliche Salze übergeführt. Als solche seien beispielsweise genannt wasserlösliche oder wasserunlösliche Säureadditionssalze, wie das Hydrobromid, Hydroiodid, Nitrat, Acetat, Benzoat, Hibenzat [2-(4-Hydroxybenzoyl)-benzoat], Fendizoat (2-[(2'-Hydroxy-4-biphenylyl)-carbonyl]-benzoat), Propionat, Butyrat, Sulfosalicylat, Laurat, Oxalat, Amsonat (4,4'-Diaminostilben-2,2'-disulfonat), Embonat [4,4'-Methylen-bis-(3-hydroxy-2-naphthoat)], Metembonat [4,4'-Methylen-bis-(3-methoxy-2-naphthoat)], Stearat, 2-Hydroxy-3-naphthoat, 3-Hydroxy-2-naphthoat, insbesondere das Hydrochlorid, Phosphat, Sulfat, Citrat, Gluconat, Maleat, Malat, Fumarat, Succinat, Tartrat, Tosilat (p-Toluolsulfonat), Mesilat (Methansulfonat), Amidosulfonat.

Eine Ausgestaltung der Erfindung sind substituierte Pyrazolobenzodiazepinone der allgemeinen Formel I*

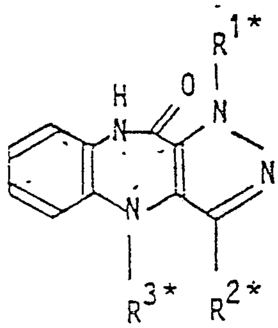

(I*),

worin

$R^{1*}$ einen Methyl- oder Ethylrest,

$R^{2*}$ ein Wasserstoffatom, einen Methyl- oder Ethylrest,

$R^{3*}$ ein Wasserstoffatom oder die Gruppe $-CO-A^*-R^{4*}$

$R^{4*}$ ein Chloratom und

A* eine geradkettige oder verzweigte Alkylengruppe mit 1 oder 2 Kohlenstoffatomen bedeuten,

und ihre Säureadditionssalze.

Bevorzugte Vertreter der Ausgestaltung I* sind solche, in denen A* eine Methylengruppe bedeutet.

Besonders bevorzugte Vertreter der Ausgestaltung I* sind solche, in denen $R^{1*}$ einen Methyl- oder Ethylrest und $R^{2*}$ ein Wasserstoffatom, einen Methyl- oder Ethylrest, $R^{3*}$ ein Wasserstoffatom oder die Gruppe $-CO-CH_2-Cl$ bedeuten, sowie ihre Säureadditionssalze.

Als Vertreter der erfindungsgemäßen Verbindungen seien beispielsweise genannt

4-[Bromacetyl]-1,3-dimethyl-1,4,9,10-tetrahydropyrazolo[4,3-b][1,5]benzodiazepin-10-on,

4-[Iodacetyl]-1,3-dimethyl-1,4,9,10-tetrahydropyrazolo[4,3-b][1,5]benzodiazepin-10-on,

4-[3-Brompropionyl]-1,3-dimethyl-1,4,9,10-tetrahydropyrazolo[4,3-b][1,5]-benzodiazepin-10-on,

4-[3-Iodpropionyl]-1,3-dimethyl-1,4,9,10-tetrahydropyrazolo[4,3-b][1,5]-benzodiazepin-10-on,

4-[Chloracetyl]-1,3-diethyl-1,4,9,10-tetrahydropyrazolo[4,3-b][1,5]-benzodiazepin-10-on,

bevorzugt

1,3-Dimethyl-1,4,9,10-tetrahydropyrazolo[4,3-b][1,5]benzodiazepin-10-on

und

4-Chloracetyl-1,3-dimethyl-1,4,9,10-tetrahydropyrazolo[4,3-b][1,5]-benzodiazepin-10-on.

Die substituierten Pyrazolobenzodiazepinone der allgemeinen Formel I und ihre Säureadditionssalze bzw. der Ausgestaltung I* besitzen wertvolle Eigenschaften, die sie gewerblich verwertbar machen. Die substituierten Pyrazolobenzodiazepinone der allgemeinen Formel I, in der $R^3$ ein Wasserstoffatom oder die Gruppe -CO-A-Hal bedeutet und A und Hal die oben angegebenen Bedeutungen haben, bzw. die der Ausgestaltung I* sind wertvolle Zwischenprodukte bei der Herstellung von pharmakologisch wirksamen und therapeutisch einsetzbaren erfindungsgemäßen Verbindungen.

Ein weiterer Gegenstand der Erfindung ist ein Verfahren zur Herstellung der substituierten Pyrazolobenzodiazepinone der allgemeinen Formel I

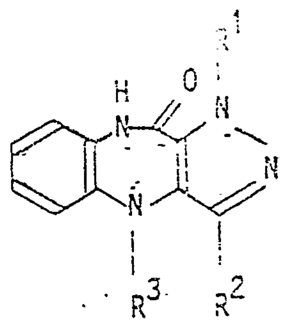 (I),

worin

$R^1$ einen Alkylrest mit 1 bis 4 Kohlenstoffatomen,

$R^2$ ein Wasserstoffatom oder einen Alkylrest mit 1 bis 4 Kohlenstoffatomen,

$R^3$ ein Wasserstoffatom oder die Gruppe $-CO-A-R^4$,

$R^4$ ein Halogenatom und

A eine geradkettige oder verzweigte Alkylengruppe mit 1 bis 5 Kohlenstoffatomen bedeuten,

und ihrer Säureadditionssalze.

Das Verfahren ist dadurch gekennzeichnet, daß man Anilinopyrazolcarbonsäurederivate der allgemeinen Formel II

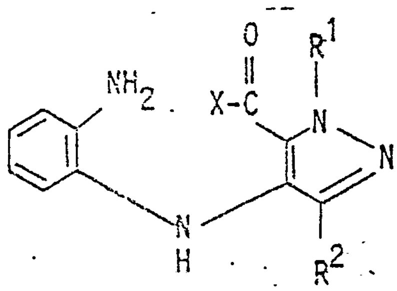 (II),

BAD ORIGINAL

worin
X eine Fluchtgruppe bedeutet,
unter Abspaltung einer Verbindung HX cyclisiert und gegebenenfalls anschließend in dem erhaltenen Reaktionsprodukt der Formel I, worin $R^3$ ein Wasserstoffatom bedeutet, das Wasserstoffatom durch eine $-CO-A-R^4$-Gruppe substituiert und/oder erhaltene Basen in die Säureadditionssalze oder erhaltene Säureadditionssalze in die freie Base oder pharmakologisch verträgliche Säureadditionssalze überführt.

Vorzugsweise stellt X eine -OH-Gruppe, eine $-O-R^9$-Gruppe oder eine $-N(R^{10})R^{11}$-Gruppe dar, worin
$R^9$ eine Alkylgruppe, eine Cycloalkylgruppe, eine gegebenenfalls substituierte Aralkylgruppe oder eine gegebenenfalls substituierte Arylgruppe und
$R^{10}$ ein Wasserstoffatom, eine Alkylgruppe, eine Cycloalkylgruppe, eine gegebenenfalls substituierte Aralkylgruppe oder eine gegebenenfalls substituierte Arylgruppe bedeuten,
$R^{11}$ eine der Bedeutungen von $R^{10}$ hat oder
$R^{10}$ und $R^{11}$ gemeinsam unter Einschluß des Stickstoffatoms, an das sie gebunden sind, einen gegebenenfalls substituierten nichtaromatischen heterocyclischen Rest bedeuten.

Als Alkylreste $R^9$, $R^{10}$ und $R^{11}$ kommen geradkettige oder verzweigte Alkylgruppen mit 1 bis 11, vorzugsweise 1 bis 5, Kohlenstoffatomen in Betracht.

Als Cycloalkylgruppen kommen solche mit 3 bis 9, vorzugsweise 5 bis 8, Kohlenstoffatomen in Betracht.

Als Aralkylgruppen kommen solche mit Arylgruppen, die bis zu 12 Kohlenstoffatome enthalten, und Alkylgruppen, die 1 bis 4 Kohlenstoffatome enthalten, in Frage, von denen die mit 6 Kohlenstoffatomen im Arylrest und 1 bis 4 Kohlenstoffatomen im Alkylrest, vor allem mit 1 Kohlenstoffatom im Alkylrest, bevorzugt sind. Beispielsweise seien genannt die Benzyl-, Phenethyl- und Phenylpropylgruppe, von denen die Benzylgruppe bevorzugt ist. Die Aralkylgruppen sind gegebenenfalls auch substituiert, von denen die im Arylrest monosubstituierten bevorzugt sind, unter anderem durch

Halogenatome, wie Fluor-, Chlor- oder Bromatome, Alkyl- und/oder Alkoxygruppen mit 1 bis 4 Kohlenstoffatomen. Beispielsweise genannt seien die 4-Chlorbenzyl-, die 3-Chlorbenzyl-, die 4-Brombenzyl-, die 2-Fluorbenzyl-, die 4-Fluorbenzyl-, die 4-Methylbenzyl-, die 4-Methoxybenzylgruppe.

Als Arylreste, die gegebenenfalls substituiert sein können, kommen solche mit 6 bis 10 Kohlenstoffatomen in Frage, beispielsweise der Phenyl- oder Naphthylrest, insbesondere der Phenylrest. Die Arylreste können ferner in beliebiger Stellung, beispielsweise mit 2, vorzugsweise mit 1 Substituenten, substituiert sein, wobei die energetisch begünstigten Stellen bevorzugt sind. Als Substituenten seien u.a. genannt Halogenatome, beispielsweise Fluor und Brom, vorzugsweise Chlor, Alkyl- oder Alkoxygruppen mit jeweils 1 bis 4 Kohlenstoffatomen. Beispiele für substituierte Arylgruppen sind die 2-Chlorphenyl-, 3-Chlorphenyl-, 4-Chlorphenyl-, 3-Bromphenyl-, 4-Bromphenyl-, 4-Fluorphenyl-, 3-Methylphenyl-, 4-Methylphenyl-, 3,4-Dichlorphenyl-, 3-Chlor-4-methylphenyl-, 4-Methoxyphenyl-, 4-Ethoxyphenyl-, 3,4-Dimethoxyphenyl-, Cumenyl- und 4-Chlor-1-naphthylgruppe, von denen die durch Halogenatome substituierten Phenylgruppen bevorzugt sind.

Aryl- und Aralkylgruppen können auch Heteroatome im Ring, beispielsweise Stickstoff-, Sauerstoff- oder Schwefelatome tragen. Beispielsweise seien genannt der 3-Pyridyl-, der 2-(1-Phenyl-4-pyrazolyl)-ethyl- und der 2-Methyl-5-benzothiazolylrest.

Als nichtaromatische heterocyclische Reste kommen solche in Betracht, die sich von gesättigten und ungesättigten Heterocyclen ableiten. Beispielsweise seien genannt die Morpholinogruppe; die Thiomorpholinogruppe; die Pyrrolidinogruppe; die Piperidinogruppe; die Piperazinogruppe; die Hexahydroazepin-1-yl-gruppe; die Hexahydro-1H-1,4-diazepin-1-yl-gruppe; eine 4-Alkyl- oder 4-Aralkylpiperazin-1-yl-gruppe, wie die 4-Methyl- oder 4-Ethyl- oder 4-Benzylpiperazin-1-yl-gruppe; eine Dialkylpiperazin-1-yl-gruppe, wie die 2,4-Dimethylpiperazin-1-yl-gruppe; eine 4-Alkyl- oder 4-Aralkyl-hexahydro-1H-1,4-diazepin-1-yl-gruppe, wie die 4-Methyl- oder 4-Ethyl- oder 4-Benzylhexahydro-1H-1,4-diazepin-1-yl-gruppe; die 1,2,3,6-Tetrahydropyrid-1-yl-gruppe.

Die Cyclisierung der Verbindungen der Formel II zu den erfindungsgemäßen Pyrazolobenzodiazepinonen der Formel I, in denen $R^3$ ein Wasserstoffatom bedeutet, wird nach an sich bekannten Verfahren durchgeführt. So erfolgt die Cyclisierung der Carbonsäuren II (X = -OH) beispielsweise durch Erwärmen, vorzugsweise in Gegenwart eines Protonen abgebenden Mittels, auf Temperaturen zwischen 90°C und 220°C, vorzugsweise 120° bis 160°C, ohne oder vorzugsweise in Gegenwart eines inerten Lösungsmittels. Als Protonen abgebende Mittel kommen anorganischen Säuren und organische Säuren, beispielsweise Halogenwasserstoffsäuren, wie Chlorwasserstoffsäure, Phosphorsäure, p-Toluolsulfonsäure oder Essigsäure in Frage. Als inerte Lösungsmittel kommen wässerige oder nicht wässerige Medien, wie Toluol, Xylol, Chlorbenzol, o-Dichlorbenzol, Trichlorbenzol oder Diphenylether in Betracht. Die Abspaltung von Wasser in nicht-wässerigem Reaktionsmedium kann gegebenenfalls mit Hilfe eines Wasserabscheiders oder durch Zusatz eines Trockenmittels, z.B. eines Molekularsiebs, erleichtert werden.

Die Cyclisierung kann auch mit Hilfe eines chemischen Kondensationsmittels vorgenommen werden, durch welches intermediär reaktive Säurederivate als Zwischenstufen, wie beispielsweise Säurehalogenide, gemischte Säureanhydride, O-Acylharnstoffe oder aktivierte Ester, gebildet werden. Als chemische Kondensationsmittel seien beispielsweise genannt Dicyclohexylcarbodiimid, Chlorameisensäureethylester, Chlorameisensäureisopropylester, Chloracetonitril, Phosphoroxidtrichlorid. Diese Cyclisierungsreaktion wird in einem inerten Lösungsmittel, beispielsweise Tetrahydrofuran, Dioxan, Dimethylformamid, N-Methylpyrrolidon bei Temperaturen von -10°C bis 120°C, vorzugsweise 0°C bis 60°C, gegebenenfalls in Gegenwart einer Hilfsbase, z.B. von wasserfreiem Alkalimetallcarbonat, Triethylamin oder Pyridin, durchgeführt.

Im Falle der Carbonsäureester II ($X = -O-R^9$) oder der Carboxamide II [$X = -N(R^{10})R^{11}$] wird die Cyclisierung bei Temperaturen zwischen 0°C und 200°C, vorzugsweise 20 und 160°C, ohne oder in Gegenwart eines inerten Lösungsmittels, gegebenenfalls in Gegenwart eines basischen oder vorzugsweise sauren Katalysators durchgeführt. Die Reaktionszeiten betragen 15 Minuten bis 4 Stunden. Als Lösungsmittel kommen beispielsweise Alkohole, wie

Ethanol oder Glykol; Ether, wie Dioxan oder Diphenylether; aromatische Kohlenwasserstoffe, wie Toluol, Xylol oder o-Dichlorbenzol; oder Dimethylsulfoxid in Frage. Als Katalysatoren seien genannt basische Katalysatoren, wie Alkalimetallalkanolate, z.B. Natriumethylat oder Kalium-tert.-butanolat, oder vorzugsweise Natriumhydrid; oder saure Katalysatoren, wie organische oder anorganische Säuren, z.B. Essigsäure, Chloressigsäure, p-Toluolsulfonsäure, o-Chlorbenzoesäure, p-Tolylsäure, Nikotinsäure, Trifluoressigsäure, Fumarsäure, Chlorwasserstoffsäure, Phosphorsäure oder Kaliumhydrogensulfat, vorzugsweise Benzoesäure, wobei bis zu 2-3 Mol saurer Katalysator je Mol Ausgangsverbindung eingesetzt werden. Wird der Aminoester der allgemeinen Formel II ($X = -O-R^9$) durch Reduktion einer Nitroverbindung der allgemeinen Formel VI hergestellt, wird bei geeigneten Reaktionsbedingungen direkt der Tricyclus der allgemeinen Formel I ($R^3 = -H$) gebildet. Bevorzugt wird die Cyclisierung mit den Aminoverbindungen II [$X = -N(R^{10})R^{11}$] durchgeführt.

Die sich gegebenenfalls anschließende Substitution erfolgt nach an sich bekannten Methoden.

Zur Herstellung der Pyrazolobenzodiazepinone der allgemeinen Formel I, in der $R^3$ eine Gruppe CO-A-Hal bedeutet, werden das erhaltene Reaktionsprodukt der Formel I, worin $R^1$ und $R^2$ die oben angegebenen Bedeutungen haben und $R^3$ ein Wasserstoffatom bedeutet, oder seine Säureadditionssalze mit Verbindungen der allgemeinen Formeln III Hal-A-CO-Hal' (III) oder IV $[Hal\text{-}A\text{-}CO]_2O$ (IV), worin Hal' eine der Bedeutungen von Hal hat und A und Hal die oben angegebenen Bedeutungen haben, umgesetzt. Diese Umsetzung wird ohne oder vorzugsweise in einem inerten Lösungsmittel bei Raumtemperatur oder erhöhter Temperatur, maximal bei der Siedetemperatur des Lösungsmittels, gegebenenfalls in Gegenwart einer Hilfsbase und/oder eines Acylierungskatalysators, vorgenommen. Die Säurehalogenide III sind gegenüber den Säureanhydriden IV bevorzugt. Als Säurehalogenid III ist Chloracetylchlorid, als Säureanhydrid IV ist Chloressigsäureanhydrid bevorzugt. Als Lösungsmittel seien beispielsweise genannt aromatische Kohlenwasserstoffe, wie Toluol, Xylol oder Chlorbenzol; offenkettige oder cyclische Ether, wie Diisopropylether oder Dioxan; chlorierte Kohlenwasserstoffe, wie Dichlorethan, andere Lösungsmittel, wie Pyridin, Acetonitril oder Dimethylformamid.

Als Hilfsbasen seien z.B. tertiäre organische Basen, wie Triethylamin und Ethyldiisopropylamin, oder Pyridin; oder anorganische Basen, wie wasserfreie Alkalimetall- oder Erdalkalimetallcarbonate oder -hydrogencarbonate oder Erdalkalimetalloxide, genannt. Als Acylierungskatalysatoren kommen beispielsweise in Frage Imidazol, Pyridin oder 4-Dimethylaminopyridin.

Das Verfahren zur Herstellung der Zwischenprodukte der allgemeinen Formel I ist also dadurch gekennzeichnet, daß man ein Anilinopyrazolderivat der allgemeinen Formel II cyclisiert und gegebenenfalls anschließend durch Verbindungen der allgemeinen Formeln III oder IV substituiert und/ oder die erhaltene Base oder deren Säureadditionssalze ineinander überführt. Bei der Herstellung der Zwischenprodukte der allgemeinen Formel I* werden entsprechende Ausgangsstoffe eingesetzt.

Säureadditionssalze erhält man durch Auflösen der erhaltenen freien Base in einem geeigneten Lösungsmittel, z.B. Wasser, Aceton, einem Alkanol, wie Ethanol oder Isopropanol, einem offenkettigen oder cyclischen Ether, wie Diethylether oder Tetrahydrofuran, das die gewünschte Säure enthält oder dem die gewünschte Säure anschließend zugegeben wird. Die Salze werden durch Filtrieren, Ausfällen mit einem Nichtlösungsmittel für das Säureadditionssalz oder durch Verdampfen des Lösungsmittels gewonnen. Salze können auch durch Oberführung in die Base und weitere Umsetzung mit einer anderen Säure in andere Salze, z.B. pharmakologisch verträgliche Säureadditionssalze, übergeführt werden.

Erhaltene Salze können z.B. durch Alkalisieren mit wäßrigem Natrium- oder Kaliumhydroxid in die freie Base umgewandelt werden, die dann durch geeignete Maßnahmen, z.B. Lösungsmittelextraktion mit einem nicht mit Wasser mischbaren Lösungsmittel, wie Chloroform, Diethylether, Toluol, gewonnen wird.

Die Herstellung der Ausgangsverbindungen der allgemeinen Formel II erfolgt durch Reduktion von Nitroverbindungen der allgemeinen Formel VI

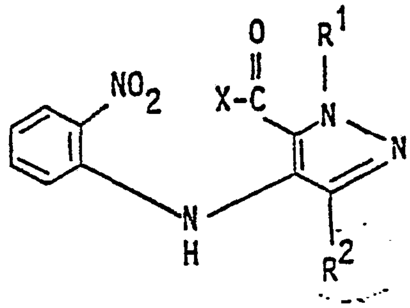

(VI),

worin $R^1$, $R^2$ und X die oben angeführte Bedeutung haben.

Die Reduktion der Nitroverbindungen erfolgt mittels üblicher Methoden, beispielsweise mit Natriumdithionit, Hydrazinhydrat und Raney-Nickel oder mit Wasserstoff in Gegenwart von Katalysatoren, wie Raney-Nickel oder Palladium auf Kohle, drucklos oder bei erhöhtem Druck, bei Raumtemperatur oder erhöhter Temperatur, in den üblichen Lösungsmitteln, wie Wasser, Alkoholen, Ethern oder Eisessig, gegebenenfalls in Gegenwart von Mineralsäuren, wie Chlorwasserstoffsäure oder Perchlorsäure.

Die Herstellung der Anilinopyrazolcarbonsäuren der allgemeinen Formel II (X = -OH) erfolgt alternativ aus den entsprechenden Säurederivaten der allgemeinen Formel II [$X = -O-R^9$ oder $-N(R^{10})R^{11}$] durch Hydrolyse im sauren oder alkalischen Milieu nach an sich bekannten Methoden.

Die Herstellung von Ausgangsverbindungen der allgemeinen Formel VI (X = -OH) wird durch Hydrolyse von Estern oder Amiden der allgemeinen Formel VI [$X = -O-R^9$ bzw. $-N(R^{10})R^{11}$] in saurem oder alkalischem Milieu unter üblichen Bedingungen vorgenommen. Vice versa erhält man die Ester oder Amide der allgemeinen Formel VI [$X = -O-R^9$ bzw. $-N(R^{10})R^{11}$] aus den Säuren der allgemeinen Formel VI (X = -OH) nach üblichen Methoden, z.B. durch Oberführung der Säure in ein Säurehalogenid und dessen Umsetzung mit $R^9$-OH, z.B. einem entsprechenden Alkohol oder Phenol bzw. dem entsprechenden Alkoholat oder Phenolat, oder einem Amin $HN(R^{10})R^{11}$.

Die Ester der allgemeinen Formel VI , insbesondere die Alkylester, bzw. die Amide der allgemeinen Formel VI [X = $-O-R^9$ bzw. $-N(R^{10})R^{11}$] werden durch Umsetzung von Aminopyrazolcarbonsäureestern der allgemeinen Formel VII bzw. Aminopyrazolcarbonsäureamiden der allgemeinen Formel VII [X = $-O-R^9$ bzw. $-N(R^{10})R^{11}$] mit o-Halogennitrobenzolen, z.B. mit o-Bromnitrobenzol unter Zusatz von Kupferkatalysatoren oder mit o-Chlornitrobenzol, vorzugsweise o-Fluornitrobenzol oder einem Gemisch aus o-Chlornitrobenzol und Fluornitrobenzol, wie es bei der Umsetzung von o-Chlornitrobenzol mit Kaliumfluorid entsteht, unter Zusatz eines Deprotonierungsmittels, z.B. Natriumhydrid, Kaliumcarbonat oder eines tertiären Amins, und gegebenenfalls eines Kronenethers, erhalten.

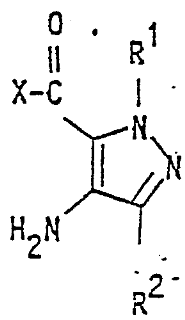 (VII) 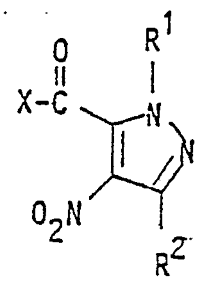 (VIII)

Die Verbindungen der allgemeinen Formel VII werden durch Reduktion von Verbindungen der allgemeinen Formel VIII nach üblichen Methoden dargestellt. Verbindungen der allgemeinen Formel VIII werden aus den entsprechenden nach literaturbekannten oder analogen Methoden hergestellten nichtnitrierten Pyrazolcarbonsäuren durch Nitrierung und gegebenenfalls deren Umwandlung in Ester oder Amide hergestellt. Unsubstituierte Carboxamide der allgemeinen Formel VIII werden durch Hydrolyse entsprechender Nitrile erhalten, die nach literaturbekannten Verfahren aus entsprechenden 5-Halogen-pyrazolen und Cyaniden hergestellt werden. Herstellungsverfahren für Ausgangsprodukte sind z.B. beschrieben in: H.A.De Wald et al., J.Med. Chem. 16, 1346(1973); US-PS 3,657,271; US-PS 3,939,161; C.Musante, Gazz. chim.ital. 75, 121-136(1945); C.A.Rojahn, Ber.Dt.Chem.Ges. 59, 607-611 (1926); L.B.Townsend et al., J.Org.Chem. 39, 2023-2027 (1974); DE-OS 22 50 316; US-PS 3,553,209.

Zur Herstellung der Verbindungen I* werden entsprechende Ausgangsverbindungen II*, III*, IV* eingesetzt.

Die folgenden Beispiele dienen zur näheren Erläuterung der Erfindung. "F." bedeutet "Schmelzpunkt"; "Kp." bedeutet "Siedepunkt"; "Zers." steht für "unter Zersetzung".

## Beispiele

### Beispiel 1

Zu 2,9 g 1,3-Dimethyl-1,4,9,10-tetrahydropyrazolo[4,3-b][1,5]benzodiazepin-10-on, 6 g wasserfreiem Kaliumcarbonat, 40 ml Dioxan und 20 ml Toluol tropft man in 30 Minuten bei 70-80°C eine Lösung von 2,1 g Chloracetylchlorid in 10 ml Toluol und rührt noch 2 Stunden bei dieser Temperatur. Man engt zur Trockne ein, kocht den Rückstand 3 mal mit je 25 ml Chloroform aus und engt das Filtrat zur Trockne ein. Man erhält 3,6 g 4-Chloracetyl-1,3-dimethyl-1,4,9,10-tetrahydropyrazolo[4,3-b][1,5]benzodiazepin-10-on, F. 246-248°C (Zers.).

### Beispiel 2

Zu einer siedenden Lösung von 2,3 g 1,3-Dimethyl-1,4,9,10-tetrahydropyrazolo[4,3-b][1,5]benzodiazepin-10-on in 30 ml absolutem Dioxan werden in 40 Minuten gleichzeitig 2 g Chloracetylchlorid und 2 g Triethylamin getropft und 3 Stunden weitergerührt. Man läßt erkalten, filtriert, engt das Filtrat zur Trockne ein und chromatographiert über eine Kieselgelsäule mittels eines Gemisches von Petrolether/Essigsäureethylester (1:1), kristallisiert aus Toluol um und erhält 2,0 g 4-Chloracetyl-1,3-dimethyl-1,4,9,10-tetrahydropyrazolo[4,3-b][1,5]benzodiazepin-10-on, F. 246-248°C (Zers.).

### Beispiel 3

Man rührt 6,7 g 1-Ethyl-3-methyl-1,4,9,10-tetrahydropyrazolo[4,3-b][1,5]-benzodiazepin-10-on, 4,0 g Chloracetylchlorid, 10 g wasserfreies Kaliumcarbonat und 70 ml trockenes Dioxan unter Stickstoff 1 Stunde bei 80°C, filtriert in der Wärme und rührt den Niederschlag der anorganischen Salze 3 mal warm mit Chloroform aus. Die organischen Filtrate werden im Vakuum zur Trockne eingeengt; der Rückstand wird mit Wasser und Natriumhydrogencarbonatlösung bei pH 6 gerührt, mit Wasser gewaschen und im Vakuum bei 50°C getrocknet. Man erhält 8,7 g 4-Chloracetyl-1-ethyl-3-methyl-1,4,9,10-tetrahydro-pyrazolo[4,3-b][1,5]benzodiazepin-10-on, F. 208-209,5°C.

Analog erhält man
4-Chloracetyl-1,3-dimethyl-1,4,9,10-tetrahydropyrazolo[4,3-b][1,5]-benzodiazepin-10-on (F. 246-248°C unter Zers.),
4-Chloracetyl-3-ethyl-1-methyl-1,4,9,10-tetrahydropyrazolo[4,3-b][1,5]-benzodiazepin-10-on (F. 217-218,5°C),
4-Chloracetyl-3-isopropyl-1-methyl-1,4,9,10-tetrahydropyrazolo[4,3-b][1,5]-benzodiazepin-10-on,
4-Chloracetyl-1-methyl-1,4,9,10-tetrahydropyrazolo[4,3-b][1,5]benzodiazepin-10-on (F. 245-246°C unter Zers.),
4-Chloracetyl-1-ethyl-1,4,9,10-tetrahydropyrazolo[4,3-b][1,5]benzodiazepin-10-on,
4-Chloracetyl-1-isopropyl-1,4,9,10-tetrahydropyrazolo[4,3-b][1,5]benzodiazepin-10-on
durch Umsetzung von Chloracetylchlorid mit
1,3-Dimethyl-1,4,9,10-tetrahydropyrazolo[4,3-b][1,5]benzodiazepin-10-on,
3-Ethyl-1-methyl-1,4,9,10-tetrahydropyrazolo[4,3-b][1,5]benzodiazepin-10-on,
3-Isopropyl-1-methyl-1,4,9,10-tetrahydropyrazolo[4,3-b][1,5]benzodiazepin-10-on,
1-Methyl-1,4,9,10-tetrahydropyrazolo[4,3-b][1,5]benzodiazepin-10-on,
1-Ethyl-1,4,9,10-tetrahydropyrazolo[4,3-b][1,5]benzodiazepin-10-on bzw.
1-Isopropyl-1,4,9,10-tetrahydropyrazolo[4,3-b][1,5]benzodiazepin-10-on.

<u>Beispiel 4</u>

Man rührt 5,0 g 1,3-Dimethyl-1,4,9,10-tetrahydropyrazolo[4,3-b][1,5]benzodiazepin-10-on, 5,2 g 3-Chlorpropionylchlorid, 11 g gemahlenes Kaliumcarbonat und 60 ml absolutes Dioxan 1,5 Stunden bei 50-60°C und destilliert das Lösungsmittel im Vakuum ab. Man versetzt den Rückstand mit Eiswasser und Salzsäure bis pH 6, läßt im Eisschrank über Nacht stehen und kristallisiert den erhaltenen Niederschlag aus Ethylacetat/Cyclohexan um. Man erhält 5,3 g 4-(3-Chlorpropionyl)-1,3-dimethyl-1,4,9,10-tetrahydropyrazolo[4,3-b][1,5]-benzodiazepin-10-on, F. 205-206,5°C (Zers.).

Analog erhält man

4-(3-Chlorpropionyl)-1-ethyl-3-methyl-1,4,9,10-tetrahydropyrazolo[4,3-b]-[1,5]benzodiazepin-10-on,

4-(3-Chlorpropionyl)-1-isopropyl-3-methyl-1,4,9,10-tetrahydropyrazolo[4,3-b]-[1,5]benzodiazepin-10-on,

4-(3-Chlorpropionyl)-3-ethyl-1-methyl-1,4,9,10-tetrahydropyrazolo[4,3-b]-[1,5]benzodiazepin-10-on,

4-(3-Chlorpropionyl)-3-isopropyl-1-methyl-1,4,9,10-tetrahydropyrazolo-[4,3-b][1,5]benzodiazepin-10-on,

4-(3-Chlorpropionyl)-1-methyl-1,4,9,10-tetrahydropyrazolo[4,3-b][1,5]benzodiazepin-10-on,

4-(3-Chlorpropionyl)-1-ethyl-1,4,9,10-tetrahydropyrazolo[4,3-b][1,5]benzodiazepin-10-on,

4-(3-Chlorpropionyl)-1-isopropyl-1,4,9,10-tetrahydropyrazolo[4,3-b][1,5]-benzodiazepin-10-on

durch Umsetzung von 3-Chlorpropionylchlorid mit

1-Ethyl-3-methyl-1,4,9,10-tetrahydropyrazolo[4,3-b][1,5]benzodiazepin-10-on,

1-Isopropyl-3-methyl-1,4,9,10-tetrahydropyrazolo[4,3-b][1,5]benzodiazepin-1o-on,

3-Ethyl-1-methyl-1,4,9,10-tetrahydropyrazolo[4,3-b][1,5]benzodiazepin-10-on,

3-Isopropyl-1-methyl-1,4,9,10-tetrahydropyrazolo[4,3-b][1,5]benzodiazepin-10-on,

1-Methyl-1,4,9,10-tetrahydropyrazolo[4,3-b][1,5]benzodiazepin-10-on,

1-Ethyl-1,4,9,10-tetrahydropyrazolo[4,3-b][1,5]benzodiazepin-10-on bzw.

1-Isopropyl-1,4,9,10-tetrahydropyrazolo[4,3-b][1,5]benzodiazepin-10-on.

Beispiel 5

Analog Beispiel 3 erhält man
4-(2-Chlorpropionyl)-1,3-dimethyl-1,4,9,10-tetrahydropyrazolo[4,3-b][1,5]-benzodiazepin-10-on,
4-(2-Chlorpropionyl)-1-ethyl-3-methyl-1,4,9,10-tetrahydropyrazolo[4,3-b]-[1,5]benzodiazepin-10-on,
4-(2-Chlorpropionyl)-1-isopropyl-3-methyl-1,4,9,10-tetrahydropyrazolo-[4,3-b][1,5]benzodiazepin-10-on,
4-(2-Chlorpropionyl)-3-ethyl-1-methyl-1,4,9,10-tetrahydropyrazolo[4,3-b]-[1,5]benzodiazepin-10-on,
4-(2-Chlorpropionyl)-3-isopropyl-1-methyl-1,4,9,10-tetrahydropyrazolo[4,3-b]-[1,5]benzodiazepin-10-on,
4-(2-Chlorpropionyl)-1-methyl-1,4,9,10-tetrahydropyrazolo[4,3-b][1,5]benzodiazepin-10-on,
4-(2-Chlorpropionyl)-1-ethyl-1,4,9,10-tetrahydropyrazolo[4,3-b][1,5]benzodiazepin-10-on,
4-(2-Chlorpropionyl)-1-isopropyl-1,4,9,10-tetrahydropyrazolo[4,3-b][1,5]-benzodiazepin-10-on
durch Umsetzung von 2-Chlorpropionylchlorid mit
1,3-Dimethyl-1,4,9,10-tetrahydropyrazolo[4,3-b][1,5]benzodiazepin-10-on,
1-Ethyl-3-methyl-1,4,9,10-tetrahydropyrazolo[4,3-b][1,5]benzodiazepin-10-on,
1-Isopropyl-3-methyl-1,4,9,10-tetrahydropyrazolo[4,3-b][1,5]benzodiazepin-10-on,
3-Ethyl-1-methyl-1,4,9,10-tetrahydropyrazolo[4,3-b][1,5]benzodiazepin-10-on,
3-Isopropyl-1-methyl-1,4,9,10-tetrahydropyrazolo[4,3-b][1,5]benzodiazepin-10-on,
1-Methyl-1,4,9,10-tetrahydropyrazolo[4,3-b][1,5]benzodiazepin-10-on,
1-Ethyl-1,4,9,10-tetrahydropyrazolo[4,3-b][1,5]benzodiazepin-10-on bzw.
1-Isopropyl-1,4,9,10-tetrahydropyrazolo[4,3-b][1,5]benzodiazepin-10-on
und
4-(4-Chlorbutyryl)-1,3-dimethyl-1,4,9,10-tetrahydropyrazolo[4,3-b][1,5]-benzodiazepin-10-on bzw.
4-(5-Chlorvaleryl)-1,3-dimethyl-1,4,9,10-tetrahydropyrazolo[4,3-b][1,5]-benzodiazepin-10-on
durch Umsetzung von 4-Chlorbutyrylchlorid bzw. 5-Chlorvalerylchlorid mit
1,3-Dimethyl-1,4,9,10-tetrahydropyrazolo[4,3-b][1,5]benzodiazepin-10-on.

Beispiel 6

Man erhitzt 7,1 g 4-[(2-Aminophenyl)amino]-N,N,1,3-tetramethyl-pyrazol-5-carboxamid, 4,7 g Benzoesäure und 8 ml trockenes Xylol 75 Minuten zum Sieden und destilliert das Lösungsmittel im Vakuum ab. Man löst den Rückstand in 70 ml Chloroform, wäscht die organische Lösung mit Natronlauge und Wasser, trocknet sie mit Magnesiumsulfat und filtriert über eine Schicht Kieselgel. Man erhält als gelbe Kristalle 5,45 g 1,3-Dimethyl-1,4,9,10-tetrahydropyrazolo[4,3-b][1,5]benzodiazepin-10-on, F. 189-190°C.

Die Cyclisierung erfolgt analog, wenn anstelle von Benzoesäure andere mittelstarke Säuren, wie o-Chlorbenzoesäure, Nicotinsäure, m-Tolylsäure, Chloressigsäure, eingesetzt werden.

Beispiel 7

Man rührt eine Schmelze aus 14,3 g 4-[(2-Aminophenyl)amino]-1-ethyl-N,N,3-trimethyl-pyrazol-5-carboxamid und 9,1 g Benzoesäure 2,5 Stunden bei 150°C, versetzt in der Wärme mit 50 ml Chloroform, rührt die Lösung mit Natriumhydrogencarbonatlösung und Wasser aus und engt sie im Vakuum ein. Der Rückstand wird über eine Kieselgelsäule mit Petrolether (Kp. 50/70°C)/Essigsäureethylester (3:7) chromatographiert. Man erhält als gelbe Kristalle 8,2 g 1-Ethyl-3-methyl-1,4,9,10-tetrahydropyrazolo[4,3-b][1,5]benzodiazepin-10-on, F. 161-162°C.

In analoger Weise erhält man
1,3-Dimethyl-1,4,9,10-tetrahydropyrazolo[4,3-b][1,5]benzodiazepin-10-on
durch Erhitzen von
4-[(2-Aminophenyl)amino]-N,N-diethyl-1,3-dimethyl-pyrazol-5-carboxamid,
4-[(2-Aminophenyl)amino]-N,1,3-trimethyl-N-phenyl-pyrazol-5-carboxamid,
1-{(4-[(2-Aminophenyl)amino]-1,3-dimethyl-pyrazol-5-yl)-carbonyl}-piperidin
oder
4-[(2-Aminophenyl)amino]-N,1,3-trimethyl-pyrazol-5-carboxamid
in Gegenwart von Benzoesäure.

Beispiel 8

Man erhitzt 8,3 g 4-[(2-Aminophenyl)amino]-1,3-dimethyl-pyrazol-5-carboxamid und 8,4 g Benzoesäure 45 Minuten auf 125°C. Die erkaltete Schmelze wird mit verdünnter Natriumhydrogencarbonatlösung gerührt; der gelbe Niederschlag wird abgesaugt und mit Wasser gewaschen. Man erhält 7,1 g 1,3-Dimethyl-1,4,9,10-tetrahydropyrazolo[4,3-b][1,5]benzodiazepin-10-on, F. 189-190°C (aus Essigsäureethylester).

Beispiel 9

Man erhitzt 2,0 g 4-[(2-Aminophenyl)amino]-1,3-dimethyl-pyrazol-5-carbonsäuremethylester und 2,0 g Benzoesäure 1 Stunde auf 120°C und anschliessend 2 Stunden auf 140°C, wonach das Dünnschichtchromatogramm vollständige Umsetzung anzeigt. Die erkaltete Schmelze wird mit warmer Natriumhydrogencarbonatlösung gerührt, der abgesaugte Niederschlag (1,55 g) wird aus Essigsäureethylester und Cyclohexan umkristallisiert. Man erhält 1,3-Dimethyl-1,4,9,10-tetrahydropyrazolo[4,3-b][1,5]benzodiazepin-10-on, F. 189-190°C.

Analog erhält man 1,3-Dimethyl-1,4,9,10-tetrahydropyrazolo[4,3-b][1,5]-benzodiazepin-10-on durch Erhitzen von
4-[(2-Aminophenyl)amino]-1,3-dimethyl-pyrazol-5-carbonsäureethylester bzw.
4-[(2-Aminophenyl)amino]-1,3-dimethyl-pyrazol-5-carbonsäure-n-amylester
in Gegenwart von Benzoesäure.

Beispiel 10

Analog Beispiel 6 erhält man

1-Isopropyl-3-methyl-1,4,9,10-tetrahydropyrazolo[4,3-b][1,5]benzodiazepin-10-on,

3-Ethyl-1-methyl-1,4,9,10-tetrahydropyrazolo[4,3-b][1,5]benzodiazepin-10-on (F. 164-165°C),

3-Isopropyl-1-methyl-1,4,9,10-tetrahydropyrazolo[4,3-b][1,5]benzodiazepin-10-on,

1-Methyl-1,4,9,10-tetrahydropyrazolo[4,3-b][1,5]benzodiazepin-10-on (F. 201-203°C),

1-Ethyl-1,4,9,10-tetrahydropyrazolo[4,3-b][1,5]benzodiazepin-10-on,

1-Isopropyl-1,4,9,10-tetrahydropyrazolo[4,3-b][1,5]benzodiazepin-10-on

wenn man in Xylol in Gegenwart von Benzoesäure
4-[(2-Aminophenyl)amino]-1-isopropyl-N,N,3-trimethyl-pyrazol-5-carboxamid,
4-[(2-Aminophenyl)amino]-3-ethyl-N,N,1-trimethyl-pyrazol-5-carboxamid,
4-[(2-Aminophenyl)amino]-3-isopropyl-N,N,1-trimethyl-pyrazol-5-carboxamid,
4-[(2-Aminophenyl)amino]-N,N,1-trimethyl-pyrazol-5-carboxamid,
4-[(2-Aminophenyl)amino]-1-ethyl-N,N-dimethyl-pyrazol-5-carboxamid bzw.
4-[(2-Aminophenyl)amino]-1-isopropyl-N,N-dimethyl-pyrazol-5-carboxamid
erhitzt.

Beispiel 11

Man hydriert 2,4 g 1,3-Dimethyl-4-[(2-nitrophenyl)amino]-pyrazol-5-carbonsäurephenylester in 150 ml Toluol und 5 ml Eisessig mit 0,75 g 10 % Palladium-Kohle bei Raumtemperatur 2,1 Stunden in einer Umlaufhydrierungsapparatur. Man engt die filtrierte Lösung im Vakuum ein und chromatographiert den Rückstand über eine Kieselgelsäule mit Petrolether ($50^{o}/70^{o}C$)/ Essigsäureethylester (1:1) und erhält 1,1 g 1,3-Dimethyl-1,4,9,10-tetrahydropyrazolo[4,3-b][1,5]benzodiazepin-10-on, F. 189-190°C.

Hydriert man den Phenylester ohne Zusatz von Eisessig, bildet sich unter langsamer Wasserstoffaufnahme 4-[(2-Aminophenyl)amino]-1,3-dimethyl-pyrazol-5-carbonsäurephenylester, der nach Zusatz von Eisessig zum Pyrazolobenzodiazepinon ringschließt.

Beispiel 12

Man versetzt 4-[(2-Aminophenyl)amino]-1,3-dimethyl-pyrazol-5-carbonsäure (Rückstand aus Versuch 4) mit 80 ml trockenem Dioxan und 8,3 g Triethylamin, tropft bei 0°C 8,8 g Chlorameisensäureethylester in 15 Minuten zu und rührt noch 1,5 Stunden unter Erwärmung auf Raumtemperatur. Man versetzt die Lösung mit Natriumhydrogencarbonatlösung, engt bis zur Trockne ein und kocht den Rückstand mit Chloroform aus. Man engt ein und chromatographiert den Rückstand über eine Kieselgelsäule mit Petrolether ($50/70^{o}C$)/ Essigester (1:1) und erhält 7,5 g 1,3-Dimethyl-1,4,9,10-tetrahydropyrazolo-[4,3-b][1,5]benzodiazepin-10-on, F. 189-190°C (aus Toluol).

Die folgenden "Versuche" dienen zur näheren Erläuterung der Herstellung der Ausgangsverbindungen (Zwischenprodukte)

A. 4-[2-(Aminophenyl)amino]-5-pyrazolcarbonsäurederivate

Versuch 1: 4-[2-(Aminophenyl)amino]-N,N,1,3-tetramethylpyrazol-5-carboxamid

85,4 g N,N,1,3-Tetramethyl-4-[(2-nitrophenyl)amino]-pyrazol-5-carboxamid, 8,5 g 10 % Palladium-Kohle und 1100 ml Methanol werden 3,5 Stunden bei 25°C in einer Umlaufhydrierungsapparatur drucklos hydriert. Man filtriert, engt die Lösung zur Trockne ein und rührt den Rückstand mit einer Mischung aus 80 ml Petrolether (Kp. 50-70°C) und 40 ml Essigsäureethylester. Man erhält 75 g 4-[2-(Aminophenyl)amino]-N,N,1,3-tetramethyl-pyrazol-5-carboxamid, zuerst F. 137-139°C, beim Stehen Umwandlung in ein Produkt mit F. 171-172°C.

Analog erhält man
4-[(2-Aminophenyl)amino]-N,1,3-trimethyl-pyrazol-5-carboxamid (F.151,5-152.5°C)
4-[(2-Aminophenyl)amino]-N,N-diethyl-1,3-dimethyl-pyrazol-5-carboxamid,
1-[{4-[(2-Aminophenyl)amino]-1,3-dimethyl-pyrazol-5-yl}-carbonyl]-piperidin,
4-[(2-Aminophenyl)amino]-1-ethyl-N,N,3-trimethyl-pyrazol-5-carboxamid (F. 144-145°C),
4-[(2-Aminophenyl)amino]-1-isopropyl-N,N,3-trimethyl-pyrazol-5-carboxamid,
4-[(2-Aminophenyl)amino]-N,N,1-trimethyl-pyrazol-5-carboxamid (F.142-144°C),
4-[(2-Aminophenyl)amino]-1-ethyl-N,N-dimethyl-pyrazol-5-carboxamid,
4-[(2-Aminophenyl)amino]-1-isopropyl-N,N-dimethyl-pyrazol-5-carboxamid,
4-[(2-Aminophenyl)amino]-3-ethyl-N,N,1-trimethyl-pyrazol-5-carboxamid,
4-[(2-Aminophenyl)amino]-3-isopropyl-N,N,1-trimethyl-pyrazol-5-carboxamid,
4-[(2-Aminophenyl)amino]-N,1,3-trimethyl-N-phenyl-pyrazol-5-carboxamid
aus
N,1,3-Trimethyl-4-[(2-nitrophenyl)amino]-pyrazol-5-carboxamid,
N,N-Diethyl-1,3-dimethyl-4-[(2-nitrophenyl)amino]-pyrazol-5-carboxamid,
1-[{1,3-Dimethyl-4-[(2-nitrophenyl)amino]-pyrazol-5-yl}-carbonyl]-piperidin,
1-Ethyl-N,N,3-trimethyl-4-[(2-nitrophenyl)amino]-pyrazol-5-carboxamid,

1-Isopropyl-N,N,3-trimethyl-4-[(2-nitrophenyl)amino]-pyrazol-5-carboxamid,
N,N,1-Trimethyl-4-[(2-nitrophenyl)amino]-pyrazol-5-carboxamid,
1-Ethyl-N,N-dimethyl-4-[(2-nitrophenyl)amino]-pyrazol-5-carboxamid,
1-Isopropyl-N,N-dimethyl-4-[(2-nitrophenyl)amino]-pyrazol-5-carboxamid,
3-Ethyl-N,N,1-trimethyl-4-[(2-nitrophenyl)amino]-pyrazol-5-carboxamid,
3-Isopropyl-N,N,1-trimethyl-4-[(2-nitrophenyl)amino]-pyrazol-5-carboxamid bzw.
N,1,3-Trimethyl-4-[(2-nitrophenyl)amido]-N-phenyl-pyrazol-5-carboxamid
durch Reduktion der Nitrogruppe.

Versuch 2: 4-[(2-Aminophenyl)amino]-1,3-dimethyl-pyrazol-5-carboxamid

Man hydriert 8,6 g 1,3-Dimethyl-4-[(2-nitrophenyl)amino]-pyrazol-5-carboxamid an 0,8 g 10 % Palladium-Kohle und 400 ml Ethanol 1 Stunde bei 50°C analog Versuch 1. Man filtriert, engt das Filtrat zur Trockne ein, kristallisiert den Eindampfungsrückstand aus 160 ml Essigsäureethylester und 120 ml Cyclohexan um und erhält 7,1 g 4-[(2-Aminophenyl)amino]-1,3-dimethyl-pyrazol-5-carboxamid. F. 182-184°C (Zers.).

Versuch 3: 4-[(2-Aminophenyl)amino]-1,3-dimethyl-pyrazol-5-carbonsäure-n-amylester

15 g 1,3-Dimethyl-4-[(2-nitrophenyl)amino]-pyrazol-5-carbonsäure-n-amylester werden in 250 ml Ethanol mit 4 g 10 % Palladium-Kohle 5 Stunden in einer drucklosen Umlaufhydrierungsapparatur bei Raumtemperatur hydriert, wonach das Dünnschichtchromatogramm quantitative Umsetzung anzeigt. Man filtriert den Katalysator ab, engt die Lösung im Vakuum ein und erhält als öligen Rückstand 13,5 g 4-[(2-Aminophenyl)amino]-1,3-dimethyl-pyrazol-5-carbonsäure-n-amylester.

Analog erhält man
4-[(2-Aminophenyl)amino]-1,3-dimethyl-pyrazol-5-carbonsäuremethylester (F. 100-101,5°C),
4-[(2-Aminophenyl)amino]-1,3-dimethyl-pyrazol-5-carbonsäureethylester,
4-[(2-Aminophenyl)amino]-1,3-dimethyl-pyrazol-5-carbonsäurephenylester
aus
1,3-Dimethyl-4-[(2-nitrophenyl)amino]-pyrazol-5-carbonsäuremethylester,
1,3-Dimethyl-4-[(2-nitrophenyl)amino]-pyrazol-5-carbonsäureethylester bzw.
1,3-Dimethyl-4-[(2-nitrophenyl)amino]-pyrazol-5-carbonsäurephenylester
durch Reduktion der Nitrogruppe (Hydrierung) in Methanol.

Versuch 4: 4-[(2-Aminophenyl)amino]-1,3-dimethyl-pyrazol-5-carbonsäure

Man versetzt 13 g 1,3-Dimethyl-4-[(2-aminophenyl)amino]-pyrazol-5-carbonsäure-n-amylester bei Raumtemperatur mit einer Lösung von 2,9 g Natriumhydroxid in 100 ml entgastem Ethanol und rührt 3 Stunden bei Raumtemperatur. Man stellt die Lösung mit verdünnter Salzsäure auf pH 9, destilliert den Alkohol ab, schüttelt die wässerige Lösung mit Dichlormethan aus, stellt die wässerige Lösung auf pH 7 und engt bei 60°C im Vakuum ein. Als Rückstand erhält man vermischt mit anorganischen Salzen zum Teil ölige 4-(2-Aminophenylamino)-1,3-dimethyl-pyrazol-5-carbonsäure.

B: 4-[(2-Nitrophenyl)amino]-pyrazol-5-carbonsäurederivate

Versuch 5: N,N,1,3-Tetramethyl-4-[(2-nitrophenyl)amino]-pyrazol-5-carboxamid

Zu einer Lösung von 26,5 g 4-Amino-N,N,1,3-tetramethylpyrazol-5-carboxamid in 100 ml Dimethylformamid fügt man unter Stickstoff 10,5 g Natriumhydrid (75 % in Paraffinöl), tropft zu der Suspension innerhalb 2 Stunden bei 40-45°C eine Lösung von 28 g 1-Fluor-2-nitrobenzol in 25 ml Dimethylformamid, rührt noch 4 Stunden bei 40°C, neutralisiert die rote Lösung durch Zugabe von Eisessig und destilliert das Lösungsmittel im Vakuum ab. Man unterwirft den Rückstand einer Wasserdampfdestillation, löst den Rückstand in Dichlormethan/Methanol (9:1), chromatographiert über Kieselgel und erhält 35,0 g gelbe Kristalle von N,N,1,3-Tetramethyl-4-[(2-nitrophenyl)amino]-pyrazol-5-carboxamid. F. 144-146°C (aus Essigsäureethylester).

Analog erhält man

N,N-Diethyl-1,3-dimethyl-4-[(2-nitrophenyl)amino]-pyrazol-5-carboxamid,

1-[{1,3-Dimethyl-4-[(2-nitrophenyl)amino]-pyrazol-5-yl}-carbonyl]-piperidin,

1-Ethyl-N,N,3-trimethyl-4-[(2-nitrophenyl)amino]-pyrazol-5-carboxamid (F. 97-98°C),

1-Isopropyl-N,N,3-trimethyl-4-[(2-nitrophenyl)amino]-pyrazol-5-carboxamid,

N,N,1-Trimethyl-4-[(2-nitrophenyl)amino]-pyrazol-5-carboxamid (F. 138,5 - 140°C),

1-Ethyl-N,N-dimethyl-4-[(2-nitrophenyl)amino]-pyrazol-5-carboxamid, 1-Isopropyl-N,N-dimethyl-4-[(2-nitrophenyl)amino]-pyrazol-5-carboxamid, 3-Ethyl-N,N,1-trimethyl-4-[(2-nitrophenyl)amino]-pyrazol-5-carboxamid (F. 114-115°C) bzw. 3-Isopropyl-N,N,1-trimethyl-4-[(2-nitrophenyl)amino]-pyrazol-5-carboxamid aus 4-Amino-N,N-diethyl-1,3-dimethyl-pyrazol-5-carboxamid, 1-[(4-Amino-1,3-dimethyl-pyrazol-5-yl)-carbonyl]-piperidin, 4-Amino-1-ethyl-N,N,3-trimethyl-pyrazol-5-carboxamid, 4-Amino-1-isopropyl-N,N,3-trimethyl-pyrazol-5-carboxamid, 4-Amino-N,N,1-trimethyl-pyrazol-5-carboxamid, 4-Amino-1-ethyl-N,N-dimethyl-pyrazol-5-carboxamid, 4-Amino-1-isopropyl-N,N-dimethyl-pyrazol-5-carboxamid, 4-Amino-3-ethyl-N,N,1-trimethyl-pyrazol-5-carboxamid bzw. 4-Amino-3-isopropyl-N,N,1-trimethyl-pyrazol-5-carboxamid durch Umsetzung mit 1-Fluor-2-nitrobenzol.

<u>Versuch 6:</u> N,N,1,3-Tetramethyl-4-[(2-nitrophenyl)amino]-pyrazol-5-carboxamid

Zu einer Lösung von 2,0 g 4-Amino-N,N,1,3-tetramethyl-pyrazol-5-carboxamid in 20 ml Dimethylformamid fügt man unter Stickstoff 1,1 g Natriumhydrid (75 % in Paraffinöl) und dann 3,5 g 1-Chlor-2-nitrobenzol, rührt 4 Stunden bei 40°C und 24 Stunden bei Raumtemperatur und arbeitet analog Versuch 5 auf. Man erhält 2,0 g N,N,1,3-Tetramethyl-4-[(2-nitrophenyl)-amino]-pyrazol-5-carboxamid, F. 144-146°C.

<u>Versuch 7:</u> N,N,1,3-Tetramethyl-4-[(2-nitrophenyl)amino]-pyrazol-5-carboxamid

2,0 g 4-Amino-N,N,1,3-tetramethyl-pyrazol-5-carboxamid, 2,0 g 1-Fluor-2-nitrobenzol und 1,8 g N-Ethylmorpholin werden 12 Stunden auf 150°C erhitzt. Man versetzt das Reaktionsprodukt mit verdünnter Salzsäure, saugt den kristallinen Rückstand ab, trocknet ihn und rührt mit wenig Petrolether (Kp. 40°C) aus. Man erhält 2,5 g N,N,1,3-Tetramethyl-4-[(2-nitrophenyl)amino]-pyrazol-5-carboxamid, F. 144-146°C (aus Essigester).

Analog erhält man aus 4-Amino-N,1,3-trimethyl-pyrazol-5-carboxamid-hydrochlorid, N-Ethylmorpholin und 1-Fluor-2-nitrobenzol als gelbe Kristalle N,1,3-Trimethyl-4-[(2-nitrophenyl)amino]-pyrazol-5-carboxamid, F. 223-225°C.

Versuch 8: 1,3-Dimethyl-4-[(2-nitrophenyl)amino]-pyrazol-5-carboxamid

Man behandelt eine Mischung aus 10,35 g 4-Amino-1,3-dimethyl-pyrazol-5-carboxamid-hydrochlorid, 22,4 g 1-Brom-2-nitrobenzol, 15 g gemahlenem Kaliumcarbonat, 2,7 g Kupfer(I)-chlorid und 55 ml Dimethylformamid zur Homogenisierung mit Ultraschall und erwärmt 1 Stunde auf 90°C. Man destilliert das Lösungsmittel im Vakuum ab und chromatographiert den Rückstand mit einer Mischung aus Methylenchlorid/Methanol (93:7) über Kieselgel und kristallisiert aus Toluol um. Man erhält 10,0 g gelbe Kristalle von 1,3-Dimethyl-4-[(2-nitrophenyl)amino]-pyrazol-5-carboxamid, F. 232-233°C.

Analog erhält man
N,1,3-Trimethyl-4-[(2-nitrophenyl)amino]-pyrazol-5-carboxamid
(F. 223-225°C)
aus
4-Amino-N,1,3-trimethyl-pyrazol-5-carboxamid-hydrochlorid
durch Umsetzung mit 1-Brom-2-nitrobenzol.

Versuch 9: 1,3-Dimethyl-4-[(2-nitrophenyl)amino]-pyrazol-5-carbonsäure-n-amylester

16 g 4-Amino-1,3-dimethyl-pyrazol-5-carbonsäureethylester, 36 g 1-Brom-2-nitrobenzol, 24 g wasserfreies Kaliumcarbonat und 150 ml n-Amylalkohol werden 12 Stunden unter langsamem Abdestillieren von Reaktionswasser über eine kurze Kolonne erhitzt, wobei 8,2 g Kupferpulver und 8,2 g Kupfer(I)-chlorid, in jeweils 4 Portionen verteilt, zugesetzt werden. Man verdünnt die abgekühlte Reaktionsmischung mit 250 ml Essigsäureethylester, filtriert die anorganischen Salze ab, engt ein und chromatographiert den erhaltenen Rückstand über eine Kieselgelsäule mit Petrolether (50/70°C)/Essigsäureethylester (4:1). Durch Umesterung mit dem Lösungsmittel während der Reaktion isoliert man als Hauptfraktion nicht den entsprechenden Ethylester, sondern 20 g öligen 1,3-Dimethyl-4-[(2-nitrophenyl)amino]-pyrazol-5-carbonsäure-n-amylester. F. 60-62°C (aus Methanol/Wasser).

Versuch 10: 1,3-Dimethyl-4-[(2-nitrophenyl)amino]pyrazol-5-carbonsäure

4,05 g 1,3-Dimethyl-4-[(2-nitrophenyl)amino]-pyrazol-5-carbonsäure-n-amylester (Versuch 9) und eine Lösung von 0,7 g Natriumhydroxid in 80 ml Ethanol werden 4 Stunden bei 40°C in einer Stickstoffatmosphäre gerührt. Man stumpft die Lösung mit Eisessig auf pH 9-10 ab, engt im Vakuum ein, versetzt den Rückstand mit Wasser, schüttelt mit Dichlormethan aus und säuert die wässerige Lösung mit verdünnter Salzsäure an. Man erhält 2,7 g 1,3-Dimethyl-4-[(2-nitrophenyl)amino]-pyrazol-5-carbonsäure, F. 253-254°C (Zers.).

Versuch 11: 1,3-Dimethyl-4-[(2-nitrophenyl)amino]pyrazol-5-carbonsäurechlorid

2,6 g 1,3-Dimethyl-4-[(2-nitrophenyl)amino]-pyrazol-5-carbonsäure und 10 ml Thionylchlorid werden 3 Stunden bei 80°C gerührt, die Lösung eingeengt und je 3 mal mit 10 ml Toluol versetzt und wieder eingeengt. Man erhält als orangefarbenes Öl 2,9 g 1,3-Dimethyl-4-[(2-nitrophenyl)amino]-pyrazol-5-carbonsäurechlorid.

Versuch 12: 1,3-Dimethyl-4-[(2-nitrophenyl)amino]-pyrazol-5-carbonsäurephenylester

Das Carbonsäurechlorid aus Versuch 11 wird mit 5 ml absolutem Dioxan und 1,6 g Natriumphenolat 30 Minuten auf 80°C erwärmt, die Lösung filtriert und eingeengt. Man versetzt mit Toluol, schüttelt mehrmals mit Wasser aus und destilliert das Lösungsmittel im Vakuum ab. Man erhält als Öl 3,0 g 1,3-Dimethyl-4-[(2-nitrophenyl)amino]-pyrazol-5-carbonsäurephenylester.

Versuch 13: 1,3-Dimethyl-[4-(2-nitrophenyl)amino]pyrazol-5-carbonsäuremethylester

Man erhitzt 13,0 g 4-Amino-1,3-dimethyl-pyrazol-5-carbonsäuremethylester, 32,0 g 1-Brom-2-nitrobenzol, 10,7 g wasserfreies Kaliumcarbonat, 8,0 g Kupfer(I)-chlorid (Zugabe in 3 Portionen) und 75 ml Dimethylformamid 9 Stunden auf 150°C, destilliert das Lösungsmittel im Vakuum ab und chromatographiert den Rückstand mit Cyclohexan/Essigsäurethylester (1:1) über Kieselgel. Man erhält 3,1 g 1,3-Dimethyl-[4-(2-nitrophenyl)amino]pyrazol-5-carbonsäuremethylester, F. 127-129°C, und als Nebenprodukt 1,3-Dimethyl-4-[(2-nitrophenyl)amino]pyrazol, F. 171-173°C.

Versuch 14: N,1,3-Trimethyl-4-[(2-nitrophenyl)amino]-N-phenyl-pyrazol-5-carboxamid

Zu einer Lösung von 2,95 g 1,3-Dimethyl-4-[(2-nitrophenyl)amino]-pyrazol-5-carbonsäurechlorid in 10 ml Dioxan tropft man 2,7 g N-Methylanilin und erwärmt 1 Stunde auf 90°C. Man engt die Lösung im Vakuum ein, versetzt mit Wasser und etwas verdünnter Salzsäure und erhält 3,1 g N,1,3-Trimethyl-4-[(2-nitrophenyl)amino]-N-phenyl-pyrazol-5-carboxamid.

Analog erhält man
N,N-Diethyl-1,3-dimethyl-4-[(2-nitrophenyl)amino]-pyrazol-5-carboxamid,
1-[{1,3-Dimethyl-4-[(2-nitrophenyl)amino]-pyrazol-5-yl}-carbonyl]-piperidin,
N,1,3-Trimethyl-4-[(2-nitrophenyl)amino]-pyrazol-5-carboxamid
durch Umsetzung des Säurechlorids mit Diethylamin, Piperidin oder Methylamin.

C: 4-Aminopyrazol-5-carbonsäurederivate

Versuch 15: 4-Amino-N,N,1,3-tetramethyl-pyrazol-5-carboxamid

19,0 g N,N,1,3-Tetramethyl-4-nitro-pyrazol-5-carboxamid in 400 ml Methanol werden in Gegenwart von 2,0 g 10 % Palladium-Kohle im Autoklaven mit 80-110 bar (8000-11000 kPa) Wasserstoffdruck 8 Stunden bei Raumtemperatur hydriert. Man filtriert, destilliert das Lösungsmittel im Vakuum ab und erhält 16,1 g kristallines 4-Amino-N,N,1,3-tetramethyl-pyrazol-5-carboxamid, F. 116-118°C (aus Ethylacetat).

Analog erhält man
4-Amino-N,1,3-trimethyl-pyrazol-5-carboxamid (Hydrochlorid F. 242-244°C),
4-Amino-1-ethyl-N,N,3-trimethyl-pyrazol-5-carboxamid (F. 81-82°C),

4-Amino-N,N-diethyl-1,3-dimethyl-pyrazol-5-carboxamid,
1-[(4-Amino-1,3-dimethyl-pyrazol-5-yl)-carbonyl]-piperidin,
4-Amino-N,N,1-trimethyl-pyrazol-5-carboxamid (F. 127-133°C),
4-Amino-1-Ethyl-N,N-dimethyl-pyrazol-5-carboxamid,

4-Amino-1-isopropyl-N,N-dimethyl-pyrazol-5-carboxamid, 4-Amino-3-ethyl-N,N,1-trimethyl-pyrazol-5-carboxamid bzw. 4-Amino-3-isopropyl-N,N,1-trimethyl-pyrazol-5-carboxamid durch Hydrierung von N,1,3-Trimethyl-4-nitro-pyrazol-5-carboxamid, 1-Ethyl-N,N,3-trimethyl-4-nitro-pyrazol-5-carboxamid, N,N-Diethyl-1,3-dimethyl-4-nitro-pyrazol-5-carboxamid, 1-[(1,3-Dimethyl-4-nitro-pyrazol-5-yl)-carbonyl]-piperidin, N,N,1-Trimethyl-4-nitro-pyrazol-5-carboxamid, 1-Ethyl-N,N-dimethyl-4-nitro-pyrazol-5-carboxamid, 1-Isopropyl-N,N-dimethyl-4-nitro-pyrazol-5-carboxamid, 3-Ethyl-N,N,1-trimethyl-4-nitro-pyrazol-5-carboxamid bzw. 3-Isopropyl-N,N,1-trimethyl-4-nitro-pyrazol-5-carboxamid.

<u>Versuch 16:</u> 4-Amino-1,3-dimethyl-pyrazol-5-carboxamid

a) Man hydriert 10,5 g 1,3-Dimethyl-4-nitro-pyrazol-5-carboxamid in 150 ml Methanol mit 1,0 g 10 % Palladium-Kohle im Autoklaven mit 50 bar Wasserstoffdruck 2 Stunden bei 70°C. Man filtriert und engt im Vakuum zur Trockne ein. Die freie Base (F. 155-157°C nach Umkristallisation aus Essigsäureethylester/Cyclohexan) wird in Ethanol gelöst und mit etherischer Chlorwasserstofflösung werden 7,4 g Hydrochlorid (F. 232-234°C unter Zers.) gefällt.

b) Zu einer Suspension von 2,0 g 1,3-Dimethyl-4-nitropyrazol-5-carboxamid und ca. 0,2 g feuchtem Raney-Nickel in 15 ml Ethanol tropft man bei 60°-75°C 1,1 ml Hydrazinhydrat, hält die Lösung 1,5 Stunden am Sieden, filtriert, engt im Vakuum zur Trockne ein und fällt mit etherischer Chlorwasserstofflösung 1,3 g Hydrochlorid aus, F 232-234°C (Zers.).

<u>Versuch 17:</u> 4-Amino-1,3-dimethyl-pyrazol-5-carbonsäureethylester

20 g 1,3-Dimethyl-4-nitro-pyrazol-5-carbonsäureethylester werden in 250 ml Ethanol mit 4 g 10 % Palladium-Kohle 5,5 Stunden in einer drucklosen Umlaufhydrierungsapparatur bei Raumtemperatur hydriert. Man engt die Lösung ein und bringt den Rückstand durch Zusatz von Petrolether (50/70°C) zur

Kristallisation. Man erhält 15,6 g 4-Amino-1,3-dimethyl-pyrazol-5-carbonsäureethylester, F. 55,5-56,5°C.

Analog erhält man
4-Amino-1,3-dimethyl-pyrazol-5-carbonsäuremethylester (F. 76-78°C, Hydrochlorid F. 183-185°C unter Zers.) bzw.
4-Amino-1,3-dimethyl-pyrazol-5-carbonsäure-n-amylester (Hydrochlorid F. 131-133°C) aus
1,3-Dimethyl-4-nitro-pyrazol-5-carbonsäuremethylester bzw.
1,3-Dimethyl-4-nitro-pyrazol-5-carbonsäure-n-amylester.

D: <u>4-Nitro-pyrazol-5-carbonsäurederivate</u>

<u>Versuch 18:</u> N,N,1,3-Tetramethyl-4-nitro-pyrazol-5-carboxamid

Man tropft eine Lösung von 40 g 1,3-Dimethyl-4-nitro-pyrazol-5-carbonsäurechlorid in 50 ml Dichlormethan bei 10-20°C unter Kühlung zu 43 g einer 40 % wässerigen Dimethylaminlösung, rührt noch 20 Minuten, macht mit Kaliumcarbonat alkalisch, trennt die organische Schicht ab und schüttelt die wässerige Phase mit Dichlormethan aus. Man trocknet die organische Lösung , engt sie ein, verreibt den Rückstand mit Petrolether (Kp. 40-70°C) und erhält 38,3 g N,N,1,3-Tetramethyl-4-nitro-pyrazol-5-carboxamid, F. 57-59,5°C, das sich beim Umkristallisieren aus Cyclohexan in ein Produkt mit F. 114-115,5°C umwandelt.

Analog werden
N,1,3-Trimethyl-4-nitro-pyrazol-5-carboxamid (F. 158-160°C),
1-Ethyl-N,N,3-trimethyl-4-nitro-pyrazol-5-carboxamid (F. 59-60,5°C),
1-[(1,3-Dimethyl-4-nitro-pyrazol-5-yl)-carbonyl]-piperidin,
N,N,1-Trimethyl-4-nitro-pyrazol-5-carboxamid (F. 104-119°C),
1-Ethyl-N,N-dimethyl-4-nitro-pyrazol-5-carboxamid,
1-Isopropyl-N,N-dimethyl-4-nitro-pyrazol-5-carboxamid,
3-Ethyl-N,N,1-trimethyl-4-nitro-pyrazol-5-carboxamid (F. 91-92°C),
3-Isopropyl-N,N,1-trimethyl-4-nitro-pyrazol-5-carboxamid bzw.
4-[(1-Ethyl-3-methyl-4-nitro-pyrazol-5-yl)-carbonyl]-morpholin,

aus den entsprechenden 4-Nitro-pyrazol-5-carbonsäurechloriden und Aminen erhalten.

Versuch 19: 1,3-Dimethyl-4-nitro-pyrazol-5-carboxamid
27,2 g 1,3-Dimethyl-4-nitro-pyrazol-5-carbonitril und 65 ml konzentrierte Schwefelsäure werden 6 Stunden bei 80°C gerührt. Man gießt auf 700 g Eis und Wasser, filtriert den Niederschlag ab, suspendiert ihn in Eiswasser und neutralisiert mit Natriumhydrogencarbonat und erhält 27,3 g 1,3-Dimethyl-4-nitro-pyrazol-5-carboxamid, F. 164-165°C.

Versuch 20: 1,3-Dimethyl-4-nitro-pyrazol-5-carbonsäureethylester
Zu 37 g 1,3-Dimethyl-4-nitro-pyrazol-5-carbonsäure werden in Portionen 44,6 g Phosphorpentachlorid zugegeben, wobei sich der Ansatz allmählich verflüssigt. Unter Rühren wird auf 100°C Badtemperatur geheizt und nach 1 Stunde Rühren im Rotationsverdampfer eingeengt, 3 mal Toluol zugegeben und wieder eingeengt. Zu dem kristallisierten Rückstand von 1,3-Dimethyl-4-nitropyrazol-5-carbonsäurechlorid fügt man unter Kühlen 37 ml absolutes Ethanol hinzu, erwärmt 1 Stunde auf 50°C, engt die Lösung im Vakuum ein, versetzt mit 300 ml Essigsäureethylester, schüttelt mit Natriumhydrogencarbonatlösung aus und engt ein. Man erhält 41,2 g 1,3-Dimethyl-4-nitro-pyrazol-5-carbonsäureethylester, F. 47-48°C.

Analog erhält man
1,3-Dimethyl-4-nitro-pyrazol-5-carbonsäuremethylester (F. 71-72°C) bzw.
1,3-Dimethyl-4-nitro-pyrazol-5-carbonsäure-n-amylester (Öl)
aus 1,3-Dimethyl-4-nitro-pyrazol-5-carbonsäurechlorid und dem entsprechenden Alkohol.

Versuch 21: 1-Methyl-4-nitro-pyrazol-5-carbonsäure
18,8 g 1-Methyl-pyrazol-5-carbonsäure (F. 227-228°C) werden in einer Mischung aus 23,5 g 100 %iger Salpetersäure und 17 ml 25 %igem Oleum 8 Stunden bei 55 - 60°C und 4,5 Stunden bei 70 - 75°C gerührt. Man gießt auf Eis, extrahiert mit Dichlormethan/Ethanol (9:1) und engt ein. Man erhält 27 g 1-Methyl-4-nitro-pyrazol-5-carbonsäure, F. 162-164°C unter Zers. (aus Ethylacetat).

Versuch 22: 3-Isopropyl-1-methyl-4-nitro-pyrazol-5-carbonsäure

a) Zu einer Lösung von 23,0 g Natrium in 290 ml absolutem Ethanol tropft man bei 6-20$^{\circ}$C unter leichtem Kühlen eine Mischung aus 148 g Oxalsäurediethylester und 86 g Aceton und läßt die kristallisierende Mischung über Nacht stehen. Man verdünnt mit 500 ml Ethanol, filtriert und erhält 185 g Natrium-5-methyl-2,4-dioxo-hexanoat.

b) Zu 180 g Natriumsalz aus (a) in 800 ml Ethanol tropft man 44,6 g Eisessig in 200 ml Ethanol und bei -3$^{\circ}$ bis 0$^{\circ}$C 52,9 g Hydrazinhydrat, destilliert das Lösungsmittel im Vakuum ab, versetzt mit Wasser und extrahiert mit Chloroform. Man destiliert das Lösungsmittel im Vakuum ab, versetzt mit Toluol, engt ein, trocknet den kristallinen Rückstand über Paraffin und turbiniert ihn in 600 ml Eiswasser. Man erhält 141 g 5-Isopropyl-~~1-methyl-~~pyrazol-3-carbonsäureethylester, F. 70,5-71$^{\circ}$C (aus Wasser).

c) 5,9 g des Esters aus (b) und 4,1 g Dimethylsulfat werden 2,5 Stunden auf 90$^{\circ}$C erwärmt, dann tropft man 18 ml 6n Natronlauge zu, rührt 2,5 Stunden bei 80$^{\circ}$C, säuert in der Wärme mit konzentrierter Salzsäure an, filtriert den Niederschlag ab und rührt ihn in Eiswasser und erhält 4,1 g 3-Isopropyl-1-methyl-pyrazol-5-carbonsäure, F. 159-160$^{\circ}$C (aus Dioxan/Cyclohexan).

d) 4 g Carbonsäure aus (c), 3,6 ml 100 %ige Salpetersäure und 4,2 ml 25 %iges Oleum werden 20 Stunden bei 60$^{\circ}$C und 48 Stunden bei Raumtemperatur gerührt. Man gießt auf Eis und erhält 3-Isopropyl-1-methyl-4-nitro-pyrazol-5-carbonsäure.

Patentansprüche (DT, FR, IT, NL, BE, CH, GB, SE, LU)

1. Substituierte Pyrazolobenzodiazepinone der allgemeinen Formel I

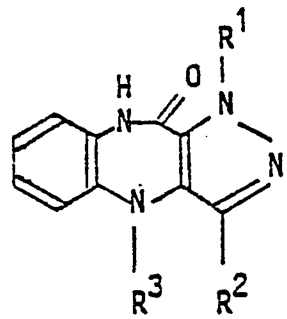

(I),

worin

$R^1$ einen Alkylrest mit 1 bis 4 Kohlenstoffatomen,

$R^2$ ein Wasserstoffatom oder einen Alkylrest mit 1 bis 4 Kohlenstoffatomen,

$R^3$ ein Wasserstoffatom oder die Gruppe $-CO-A-R^4$,

$R^4$ ein Halogenatom und

A eine geradkettige oder verzweigte Alkylengruppe mit 1 bis 5 Kohlenstoffatomen bedeuten,

und ihre Säureadditionssalze.

2. Substituierte Pyrazolobenzodiazepinone der allgemeinen Formel I nach Anspruch 1, worin

$R^1$ einen Methylrest,

$R^2$ einen Methylrest,

$R^3$ ein Wasserstoffatom oder die Gruppe $-CO-A-R^4$,

$R^4$ ein Halogenatom und

A eine geradkettige Alkylengruppe mit 1 oder 2 Kohlenstoffatomen bedeuten,

und ihre Säureadditionssalze.

3. Substituierte Pyrazolobenzodiazepinone nach Anspruch 1, gekennzeichnet durch die allgemeine Formel I*

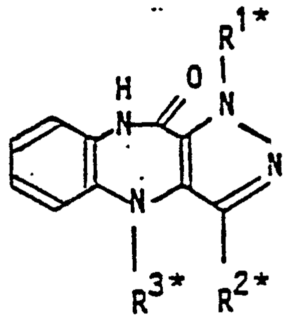

(I*),

worin

$R^{1*}$ einen Methyl- oder Ethylrest,

$R^{2*}$ ein Wasserstoffatom, einen Methyl- oder Ethylrest,

$R^{3*}$ ein Wasserstoffatom oder die Gruppe $-CO-A^*-R^{4*}$

$R^{4*}$ ein Chloratom und

A* eine geradkettige oder verzweigte Alkylengruppe mit 1 oder 2 Kohlenstoffatomen bedeuten,

und ihre Säureadditionssalze.

4. Verbindungen nach Anspruch 3, in denen A* eine Methylengruppe bedeutet.

5. Verbindungen nach Anspruch 3, in denen $R^{1*}$ einen Methyl- oder Ethylrest und $R^{2*}$ ein Wasserstoffatom, einen Methyl- oder Ethylrest, $R^{3*}$ ein Wasserstoffatom oder die Gruppe $-CO-CH_2-Cl$ bedeuten, und ihre Säureadditionssalze.

6. Verfahren zur Herstellung der substituierten Pyrazolobenzodiazepinone der allgemeinen Formel I

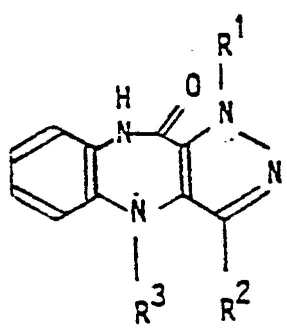

(I),

worin

$R^1$ einen Alkylrest mit 1 bis 4 Kohlenstoffatomen,

$R^2$ ein Wasserstoffatom oder einen Alkylrest mit 1 bis 4 Kohlenstoffatomen,

$R^3$ ein Wasserstoffatom oder die Gruppe $-CO-A-R^4$,

$R^4$ ein Halogenatom und

A eine geradkettige oder verzweigte Alkylengruppe mit 1 bis 5 Kohlenstoffatomen bedeuten,

und ihrer Säureadditionssalze, dadurch gekennzeichnet, daß man Anilinopyrazol-carbonsäurederivate der allgemeinen Formel II

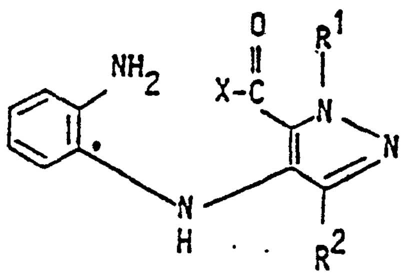

(II),

worin

X eine Fluchtgruppe bedeutet,

cyclisiert und gegebenenfalls anschließend in dem erhaltenen Reaktionsprodukt der Formel I, worin $R^3$ ein Wasserstoffatom bedeutet, das Wasserstoffatom durch eine $-CO-A-R^4$-Gruppe substituiert und/oder erhaltene Basen in die Säureadditionssalze oder erhaltene Säureadditionssalze in die freie Base oder pharmakologisch verträgliche Säureadditionssalze überführt.

7. Verfahren nach Anspruch 6, dadurch gekennzeichnet, daß man Verbindungen II einsetzt, in denen X eine -OH-Gruppe, eine $O-R^9$-Gruppe oder eine $-N(R^{10})R^{11}$-Gruppe darstellt, worin

$R^9$ eine Alkylgruppe, eine Cycloalkylgruppe, eine gegebenenfalls substituierte Aralkylgruppe oder eine gegebenenfalls substituierte Arylgruppe und

$R^{10}$ ein Wasserstoffatom, eine Alkylgruppe, eine Cycloalkylgruppe, eine gegebenenfalls substituierte Aralkylgruppe oder eine gegebenenfalls substituierte Arylgruppe bedeuten,

$R^{11}$ eine der Bedeutungen von $R^{10}$ hat oder

$R^{10}$ und $R^{11}$ gemeinsam unter Einschluß des Stickstoffatoms, an das sie gebunden sind, einen gegebenenfalls substituierten nichtaromatischen heterocyclischen Rest bedeuten.

8. Verfahren nach Anspruch 7, dadurch gekennzeichnet, daß man Verbindungen II einsetzt, in denen
$R^1$ und $R^2$ eine Methylgruppe und
X eine -OH-Gruppe oder eine $-O-R^9$-Gruppe darstellen, worin
$R^9$ eine Alkylgruppe, eine gegebenenfalls substituierte Aralkylgruppe oder eine gegebenenfalls substituierte Arylgruppe bedeutet.

9. Verfahren nach Anspruch 6, dadurch gekennzeichnet, daß man die sich gegebenenfalls anschließende Substitution mit Verbindungen der allgemeinen Formeln Hal-A-CO-Hal' (III) oder $(Hal-A-CO)_2O$ (IV), worin Hal und Hal' ein Halogenatom und A eine Alkylengruppe mit 1 bis 5 Kohlenstoffatomen bedeuten, durchführt.

Patentansprüche (AT)

1. Verfahren zur Herstellung der substituierten Pyrazolobenzodiazepinone der allgemeinen Formel I

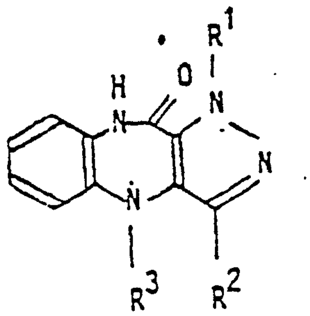

(I),

worin

$R^1$ einen Alkylrest mit 1 bis 4 Kohlenstoffatomen,

$R^2$ ein Wasserstoffatom oder einen Alkylrest mit 1 bis 4 Kohlenstoffatomen,

$R^3$ ein Wasserstoffatom oder die Gruppe $-CO-A-R^4$,

$R^4$ ein Halogenatom und

A eine geradkettige oder verzweigte Alkylengruppe mit 1 bis 5 Kohlenstoffatomen bedeuten,

und ihrer Säureadditionssalze, dadurch gekennzeichnet, daß man Anilinopyrazol-carbonsäurederivate der allgemeinen Formel II

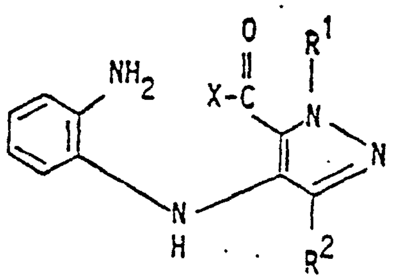

(II),

worin

X eine Fluchtgruppe bedeutet,

cyclisiert und gegebenenfalls anschließend in dem erhaltenen Reaktionsprodukt der Formel I, worin $R^3$ ein Wasserstoffatom bedeutet, das Wasserstoffatom durch eine $-CO-A-R^4$-Gruppe substituiert und/oder erhaltene Basen in die Säureadditionssalze oder erhaltene Säureadditionssalze in die freie Base oder pharmakologisch verträgliche Säureadditionssalze überführt.

2. Verfahren nach Anspruch 1, dadurch gekennzeichnet, daß man Verbindungen II einsetzt, in denen X eine -OH-Gruppe, eine O-$R^9$-Gruppe oder eine -N($R^{10}$)$R^{11}$-Gruppe darstellt, worin
$R^9$ eine Alkylgruppe, eine Cycloalkylgruppe, eine gegebenenfalls substituierte Aralkylgruppe oder eine gegebenenfalls substituierte Arylgruppe und
$R^{10}$ ein Wasserstoffatom, eine Alkylgruppe, eine Cycloalkylgruppe, eine gegebenenfalls substituierte Aralkylgruppe oder eine gegebenenfalls substituierte Arylgruppe bedeuten,
$R^{11}$ eine der Bedeutungen von $R^{10}$ hat oder
$R^{10}$ und $R^{11}$ gemeinsam unter Einschluß des Stickstoffatoms, an das sie gebunden sind, einen gegebenenfalls substituierten nichtaromatischen heterocyclischen Rest bedeuten.

3. Verfahren nach Anspruch 1, dadurch gekennzeichnet, daß man Verbindungen II einsetzt, in denen
$R^1$ und $R^2$ eine Methylgruppe und
X eine -OH-Gruppe oder eine -O-$R^9$-Gruppe darstellen, worin
$R^9$ eine Alkylgruppe, eine gegebenenfalls substituierte Aralkylgruppe oder eine gegebenenfalls substituierte Arylgruppe bedeutet.

4. Verfahren nach Anspruch 1, dadurch gekennzeichnet, daß man die sich gegebenenfalls anschließende Substitution mit Verbindungen der allgemeinen Formeln Hal-A-CO-Hal' (III) oder $(Hal\text{-}A\text{-}CO)_2O$ (IV), worin Hal und Hal' ein Halogenatom und A eine Alkylengruppe mit 1 bis 5 Kohlenstoffatomen bedeuten, durchführt.

5. Verfahren nach Anspruch 4, dadurch gekennzeichnet, daß man Verbindungen der allgemeinen Formel III oder IV, in denen A eine Methylen- oder Ehtylengruppe bedeutet, einsetzt.

6. Verfahren nach Anspruch 1, 2 oder 4, dadurch gekennzeichnet, daß man substituierte Pyrazolobenzodiazepinone der allgemeinen Formel I*

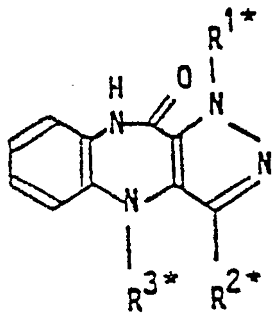 (I*),

worin

$R^{1*}$ einen Methyl- oder Ethylrest,

$R^{2*}$ ein Wasserstoffatom, einen Methyl- oder Ethylrest,

$R^{3*}$ ein Wasserstoffatom oder die Gruppe $-CO-A^*-R^{4*}$

$R^{4*}$ ein Chloratom und

$A^*$ eine geradkettige oder verzweigte Alkylengruppe mit 1 oder 2 Kohlenstoffatomen bedeuten,

oder ihre Säureadditionssalze herstellt.

7. Verfahren nach Anspruch 2, dadurch gekennzeichnet, daß man eine Verbindung II einsetzt, in der $R^1$ und $R^2$ eine Methylgruppe, X eine $-N(R^{10})R^{11}$-Gruppe und $R^{10}$ und $R^{11}$ ein Wasserstoffatom bedeuten.

8. Verfahren nach Anspruch 3, dadurch gekennzeichnet, daß man eine Verbindung II einsetzt, in der X eine $-O-R^9$-Gruppe und $R^9$ eine Alkylgruppe bedeuten.

9. Verfahren nach Anspruch 3 und 4, dadurch gekennzeichnet, daß man Chloracetylchlorid als Verbindung III einsetzt.

0064775

Europäisches Patentamt

# EUROPÄISCHER RECHERCHENBERICHT

Nummer der Anmeldung

EP 82 10 5942

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
| A | DE-A-2 707 270 (HOECHST AG) *Insgesamt* ----- | 1 | C 07 D 487/04<br>A 61 K 31/55<br>C 07 D 231/38<br>C 07 D 231/16<br>C 07 D 231/14<br>C 07 D 59/185<br>(C 07 D 487/04<br>C 07 D 243/00<br>C 07 D 231/00 |
| | | | RECHERCHIERTE SACHGEBIETE (Int. Cl. 3) |
| | | | C 07 D 487/00 |

Der vorliegende Recherchenbericht wurde für alle Patentansprüche erstellt.

| Recherchenort | Abschlußdatum der Recherche | Prüfer |
|---|---|---|
| DEN HAAG | 23-08-1982 | ALLARD M.S. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82